# EUROPÄISCHE PATENTSCHRIFT

(11) **EP 2 106 760 B1**
(45) Veröffentlichungstag und Bekanntmachung des Hinweises auf die Patenterteilung: **20.10.2010**
(21) Anmeldenummer: 09156432.8
(22) Anmeldetag: 27.03.2009
(51) Int. Cl.: A61B 17/29, A61B 17/00

(54) **Medizinisches Instrument mit einer Rastsperre**
Medicinal instrument with a break block
Instrument médical doté d'une fermeture à cran d'arrêt

(30) Priorität: 31.03.2008 DE 102008017299
(43) Veröffentlichungstag der Anmeldung: 07.10.2009
(73) Patentinhaber: Karl Storz GmbH & Co. KG, 78532 Tuttlingen (DE)
(72) Erfinder: Bacher, Uwe, 78532, Tuttlingen (DE); Summerer, Sabine, 78532, Tuttlingen (DE); Frey, Sebastian, 78054, Villingen-Schwenningen (DE)
(74) Vertreter: Witte, Weller & Partner

(56) Entgegenhaltungen:
- DE-A1- 10 353 552
- DE-T2- 69 203 964
- DE-U1-202005 020 964
- US-A- 5 626 608
- US-A- 5 827 323
- US-A1- 2007 299 469

## Beschreibung

Die Erfindung betrifft ein medizinisches Instrument, mit einem Schaft, der proximal mit einer Handhabe verbunden ist, mit einem längs des Schaft führbaren Einsatzes, der proximalseitig mit einem beweglichen Griffteil der Handhabe verbunden ist und der distalseitig ein Werkzeug aufweist, das durch Verschwenken des Griffteiles betätigbar ist, und mit einer Raste, die mit dem beweglichen Griffteil in rastenden Eingriff bringbar ist.

Ein derartiges medizinisches Instrument in Form einer Präparier- oder Fasszange ist aus dem Katalog der Anmelderin Laparoskopie, 5. Ausgabe 1/2005 Kapitel 4 "Präparier- und Fasszangen", Seite 77 bekannt.

Mittels der Raste kann das bewegliche Griffteil, das das Werkzeug betätigt, fixiert werden. Die Raste besteht meist aus einem stabförmigen Griffteil, das an einer Handhabe, meist einem unbeweglichen Griffteil angebracht ist. Die Raste weist auf der Seite, die dem beweglichen Griffteil zugewandt ist Zähne auf, die mit einem Raststift am beweglichen Griffteil zusammenwirken.

Bei manchen Ausgestaltungen ist die Neigung der Flanke der Zähne derart, dass das bewegliche Griffteil in einer Richtung die Zähne überlaufen kann, eine Bewegung in die entgegengesetzte Richtung aber gesperrt ist.

Ein häufiger praktischer Anwendungsfall ist eine Fasszange, bei der, nach Erfassen eines Gewebeabschnittes das bewegliche Griffteil mittels der Raste verrastet wird, so dass die Maulteile in einer bestimmten Stellung gehalten werden. Bei manchen Ausführungen ist, wie zuvor erwähnt, es dennoch möglich das bewegliche Griffteil in einer Richtung, meist in die schließende Stellung der Maulteile, zu bewegen, so dass das gehaltene Gewebe noch fester gehalten wird als zuvor. Eine Bewegung in die entgegengesetzte Richtung, also ein weiteres Öffnen der Maulteile ist jedoch durch die Raste gesperrt. Dadurch ist sichergestellt, dass ein einmal ergriffenes Gewebe nicht mehr freigegeben wird, so dass sich die Handhabungsperson auf andere Vorgänge, die sie mit der Zange durchführen möchte, bspw. ein Koagulieren oder dergleichen konzentrieren kann.

Möchte die Handhabungsperson die Raste lösen, muss sie diese von dem Griffteil wegbewegen und kann erst dann wieder das bewegliche Griffteil in beiden Richtungen bewegen. Da die hebelartige Raste aber oftmals durch Federkraft vorgespannt ist, muss die Handhabungsperson die Raste, wenn sie das zweite Griffteil frei hin- und herbewegen will, wegbewegen.

Dies ist ergonomisch ungünstig und belegt zumindest einen Finger der Hand, der die Raste dauernd wegrücken muss.

Bei medizinischen Instrumenten mit abwinkelbarem Schaft ist am Instrument ein weiteres Steuerelement vorhanden, nämlich das Steuerelement über das der Schaft abgewinkelt wird.

Bei manchen Konstruktionen, insbesondere bei Präparierzangen, ist es wünschenswert, dass das bewegliche Griffteil für manche Manipulationen über eine Raste fixiert ist, bei anderen Manipulationen, bspw. beim Schneiden, aber frei in beide Richtungen hin und her bewegbar ist. Da bei einem Instrument mit abgewinkeltem Schaft auch in abgewinkeltem Zustand präpariert werden soll, muss die Handhabungsperson mehrere Stellelemente bedienen können und deren Arretierung oder Freigabe steuern können.

Da solche medizinischen Instrumente meist bei der minimal-invasiven Chirurgie eingesetzt werden, ist das Sichtfeld der Handhabungsperson am distalen Ende ohnehin eingeschränkt, sie muss diesen Bereich entweder über ein Endoskop oder einen abseits liegenden Monitor beobachten, so dass es sehr wichtig ist, dass sie mit den Fingern der Hand, die das medizinische Instrument ergriffen hat, möglichst ergonomisch die unterschiedlichen Steuerzustände erreichen kann.

Es ist daher Aufgabe der vorliegenden Erfindung ein medizinisches Instrument, insbesondere solche mit abwinkelbarem Schaft, dahingehend weiterzuentwickeln, dass eine funktionssichere und ergonomische Handhabung möglich ist, insbesondere was die Arretierung bzw. die Lösung der Arretierung des beweglichen Griffteiles betrifft.

Erfindungsgemäß die Aufgabe dadurch gelöst, dass eine Rastsperre vorhanden ist, die zwischen Raste und Griffteil bringbar ist, so dass die Rastfunktion durch die Rastsperre aufhebbar ist.

Diese Maßnahme hat den Vorteil, dass, falls die Handhabungsperson für einen gewissen Zeitraum oder dauernd die Rastfunktion aufheben möchte, sie einfach die Rastsperre zwischen die Raste und das Griffteil bringt, so dass er dann mit dem beweglichen Griffteil uneingeschränkt die erforderlichen Bewegungen durchführen kann, bspw. durch ein Hin- und Herbewegen einen Präpariervorgang, bspw. ein Schneiden von Gewebe durch Öffnen und Schließen von Maulteilen, durchführen kann.

Sie braucht nun nicht mehr Sorge dafür zu tragen, die Raste von dem Eingriff mit dem beweglichen Griffteil wegzubewegen und dort zu halten, dies erfolgt erfindungsgemäß dadurch, dass die Rastsperre zwischen die Raste und das bewegliche Griffteil gebracht wird. Nachdem die Handhabungsperson die Rastsperre zwischen die Raste und das bewegliche Griffteil gebracht hat, braucht sie auch keine Aufmerksamkeit dahingehend aufzubringen, ob die Rastfunktion doch wieder einsetzen könnte. Dies wäre sehr störend, wenn sie bspw. einen sehr komplizierten oder an einem lebenswichtigen Organ, wie bspw. dem Herzen durchzuführenden Schnitt anbringen soll, der plötzlich durch eine ungewollte Rastbewegung abrupt gestoppt werden würde.

Somit ist diese Rastfunktion auf sehr sichere Weise aufhebbar und dies ist auch deswegen ergonomisch, da nur ein einziger Vorgang notwendig ist, nämlich das Verbringen der Rastsperre zwischen die Raste und das bewegliche Griffteil.

In einer weiteren Ausgestaltung der Erfindung ist die Rastsperre aus einer ersten Stellung, in der die Rastfunktion aktiviert, ist in eine zweite Stellung bringbar, in der die Rastfunktion deaktiviert ist.

Diese Maßnahme hat den Vorteil, dass die Handhabungsperson ohne all zu große Aufmerksamkeit das Umschalten der Rastsperre zwischen einer definierten ersten Stellung, in der die Rastfunktion aktiviert ist, in die zweite, die Rastfunktion deaktivierende Stellung, verbringen kann. Dasselbe gilt für den umgekehrten Vorgang.

In einer weiteren Ausgestaltung der Erfindung ist die Rastsperre in der ersten Stellung haltbar.

In einer weiteren Ausgestaltung ist die Rastsperre auch in der zweiten Stellung haltbar.

Diese Maßnahmen haben den Vorteil, dass im Hinblick auf die Funktionssicherheit die Rastsperre in diesen beiden Stellungen gehalten ist, so dass kein ungewolltes Lösen stattfindet und sich die Rastsperre in eine andere Stellung begibt.

In einer weiteren Ausgestaltung der Erfindung ist die Rastsperre in einer Führung führbar.

Diese Maßnahme hat den Vorteil, dass das Umschalten der Rastsperre zwischen den beiden Stellungen durch die Führung geführt wird und somit sicher und ergonomisch durchführbar ist. Ergonomisch deswegen, da die Handhabungsperson keine Aufmerksamkeit für den Bewegungsweg aufbringen muss, dies wird durch die Führung bewerkstelligt, die einzige Aufmerksamkeit ist dahingehend aufzuwenden, dass die Rastsperre aus der einen in die andere Stellung verbracht wird.

In einer weiteren Ausgestaltung der Erfindung weist die Führung zumindest eine Nut auf, in die ein vorspringendes Element der Rastsperre eingreift.

Diese Maßnahme hat den Vorteil, dass die Führung fertigungstechnisch sehr einfach durchzuführen ist und eine funktionssichere Führung sicherstellt.

In einer weiteren Ausgestaltung der Erfindung ist die Rastsperre in dem Bereich, in dem diese mit den Zähnen der Raste in Kontakt tritt, abgerundet ausgebildet.

Diese Maßnahme hat den Vorteil, dass die Rastsperre relativ sanft über die Zähne gleiten kann und sich zwischen das bewegliche Griffteil und die Zähne der Rasten verschieben bzw. bringen lässt. Dadurch ist auch sichergestellt, dass die Zähne nicht beeinträchtigt werden, so dass diese, wenn die Rastfunktion wieder erwünscht ist, diese sicher erfüllen können.

In einer weiteren Ausgestaltung der Erfindung ist die Rastsperre am beweglichen Griffteil angebracht.

Diese Maßnahme hat in ergonomischer Hinsicht den Vorteil, dass die Rastsperre bspw. mit dem Finger, der das Griffteil erfasst oder bewegt, in die beiden Stellungen verbracht werden kann.

In einer weiteren Ausgestaltung der Erfindung ist die Rastsperre am Griffteil verschiebbar angebracht, und ist dort zwischen der ersten und der zweiten Stellung verschiebbar.

Diese Maßnahme hat, wie zuvor erwähnt, ergonomisch den Vorteil, dass die Raste durch einen einfachen Verschiebevorgang am Griffteil, in die unterschiedlichen Positionen verschoben werden kann.

In einer weiteren Ausgestaltung der Erfindung weist das Griffteil eine Fingeröffnung auf, an deren äußerem Ringabschnitt die Rastsperre angebracht ist.

Diese Maßnahme hat den Vorteil, dass die Rastsperre entweder mit dem Finger, der in dem Ringabschnitt eingeschoben wird, oder dem nächst danebengelegenen Finger, längs der Außenseite des Ringabschnittes der Fingeröffnung, verschoben werden kann.

Dies ist sowohl funktionssicher als auch ergonomisch durchführbar.

In einer weiteren Ausgestaltung der Erfindung ist die Rastsperre als gebogenes Element ausgebildet, dessen Krümmung dem Ringabschnitt des Griffteiles angepasst ist. Dies erlaubt eine besonders ergonomische Verschiebung der Rastsperre längs der Außenseite des Ringabschnittes des Griffteiles.

In einer weiteren Ausgestaltung der Erfindung weist das gebogene Element Merkmale auf, die dessen Griffigkeit erhöhen.

Diese Merkmale haben zum einen den Vorteil, dass sie der Handhabungsperson auch ohne Sichtkontakt das Gefühl vermitteln, dass er nunmehr die Rastsperre berührt und gleichzeitig wird durch die höhere Griffigkeit sichergestellt, dass die Handhabungsperson beim Verschiebevorgang nicht von der Rastsperre abrutscht.

In einer weiteren Ausgestaltung der Erfindung sind die Merkmale ausgewählt aus Erhebungen, Riffelungen, Aussparungen, Ausstanzungen und Mulden.

Diese Maßnahme hat den Vorteil, dass diese Merkmale zum einen fertigungstechnisch sehr einfach bewerkstelligbar sind und andererseits ein sehr gutes Gefühl der Griffigkeit der Handhabungsperson vermitteln.

In einer weiteren Ausgestaltung der Erfindung stehen von der Rastsperre Führungsstifte vor, die in jeweils eine Nut an den Seiten des Ringabschnittes eingreifen.

Diese Maßnahme hat den Vorteil, dass eine sichere Führung der Rastsperre an dem Ringabschnitt gewährleistet ist.

In einer weiteren Ausgestaltung weist die Rastsperre seitliche Abdeckungen auf, die den Ringabschnitt seitlich übergreifen.

Diese Maßnahme hat den Vorteil, dass die Führung durch die Abdeckungen abgedeckt ist, und somit von dem Finger der Betätigungsperson nicht erfasst werden kann. Durch die Abdeckung können auch die zuvor erwähnten Führungsstifte gehalten werden.

In einer weiteren Ausgestaltung der Erfindung erstreckt sich die Rastsperre entlang der Außenseite des Ringabschnittes.

Diese Maßnahme hat den Vorteil, dass eine ergonomisch sehr günstige Position für die Rastsperre geschaffen ist, die sehr einfach ertastbar ist, so dass die Handhabungsperson ohne größere Aufmerksamkeit die Rastsperre betätigen kann. Die Tatsache, dass die Rastsperre entlang der Außenseite des Ringabschnittes angeordnet ist, eröffnet die Möglichkeit den Finger, der in die innere Öffnung des Ringabschnittes eingelegt ist dort zu belassen und die Steuerung der Rastsperre mit einem anderen Finger durchzuführen. Alternativ kann der Finger, der in dem Ringabschnitt eingesteckt ist, zunächst herausbewegt werden und dann ganz gezielt durch diesen Finger die Rastsperre bewegt werden.

In einer weiteren Ausgestaltung der Erfindung sind an der Rastsperre Clip-Elemente angeordnet, die in der ersten bzw. zweiten Stellung mit Haltestiften in Verbindung treten.

Diese Maßnahme hat den Vorteil, dass die Halterung der Rastsperre in beiden Stellungen zum einen durch sehr einfache Elemente bewerkstelligt wird und für die Handhabungsperson durch den Clipsvorgang auch haptisch erfassbar ist, denn sie spürt dieses Einrasten der Clip-Elemente.

In einer weiteren Ausgestaltung der Erfindung tritt In der zweiten Stellung das eine Clip-Element mit dem Raststift in haltende Verbindung, der mit der Raste zusammenwirkt.

Dies hat zum einen den Vorteil, dass dieser Raststift eine Doppelfunktion ausführt, einmal als Haltestift für die Rastsperre und andererseits als Rastelement für die Zähne der Raste. Darüber hinaus ist durch diese Maßnahme besonders einfach sichergestellt, dass dieser Raststift nicht mehr mit der Raste In Berührung treten kann, denn er ist nunmehr mit dem Clip-Element der Rastsperre belegt.

In einer weiteren Ausgestaltung der Erfindung sind die Clip-Elemente in einer Stufe angeordnet, die in einer im beweglichen Griffteil ausgesparten umfänglichen Nut eingreift.

Diese Maßnahme hat den Vorteil, dass diese Clip-Elemente durch die Rastsperre abgedeckt im äußeren Umfangsbereich des Griffteiles aufgenommen sind, somit dort geschützt sind auch vor Verschmutzungen und dergleichen, somit für eine funktionssichere Ausgestaltung sorgen.

In einer weiteren Ausgestaltung der Erfindung ist die Raste als in Richtung des beweglichen Griffteils vorgespannter verschwenkbarer Hebel ausgebildet, der an der Handhabe angelegt ist.

Diese Maßnahme hat den Vorteil, dass, falls die Rastfunktion gewünscht ist, diese sich von selbst wieder einstellt, wenn die Rastsperre aus der Position zwischen dem Griffteil und der Raste wegbewegt ist. Durch die Vorspannung wird die Raste bzw. deren Zähne an das bewegliche Griffteil gedrückt, so dass dann dort mit dem entsprechenden Rastmerkmal, also bspw. dem zuvor erwähnten Raststift, die Verrastung wieder stattfindet.

In einer weiteren Ausgestaltung der Erfindung weist die Raste einen Ansatz auf, über den die Raste von dem beweglichen Griffteil abhebbar ist.

Diese Maßnahme hat den Vorteil, dass über den Ansatz die Raste, die meist als Stab oder leistenartiges Gebilde ausgebildet ist, einfach von dem beweglichen Griffteil abhebbar ist.

Dies ermöglicht der Handhabungsperson kurzfristig die Rastfunktion aufzuheben, bspw. um nur eine einzige Bewegungsrichtung kurzzeitig durchführen zu können und anschließend durch loslassen sofort wieder die Rastfunktion zu aktivieren, ohne dass er dabei die Rastsperre einschieben muss.

In einer weiteren Ausgestaltung der Erfindung ist der Ansatz bogenförmig ausgebildet, in den ein Finger einer Hand, die die Handhabe ergriffen hat, legbar ist.

Diese Maßnahme hat nun in ergonomischer Hinsicht den besonderen Vorteil, dass auch dieser Bewegungsvorgang sicher und einfach durchführbar ist.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in den Zeichnungen 1, 2, 3, 13, 13a, 17-20 dargestellt und werden in der nachfolgenden Beschreibung näher erläutert.

Die Zeichnungen 1-16 beschreiben noch weitere Merkmale des Instruments, welche jedoch nicht Teil der Erfindung sind.

Es zeigen:
- Fig. 1: ein medizinisches Instrument mit abwinkelbaren Schaft in Seitenansicht,
- Fig. 1a: das medizinische Instrument von Fig. 1 aus proximaler Betrachtung, d.h. aus Sicht des Operateurs,
- Fig. 1b: eine perspektivische Ansicht des medizinischen Instrumentes,
- Fig. 2: eine ausschnittsweise Seitenansicht des medizinischen Instruments von Fig. 1 von der gegenüberliegenden Seite,
- Fig. 3: eine ausschnittsweise Seitenansicht des medizinischen Instruments von Fig. 1 mit geöffnetem Gehäuse einer Handhabe,
- Fig. 4: eine ausschnittsweise Detailansicht der Handhabe zur Erläuterung der Arretierung einer Abwinkelsteuerung,
- Fig. 4a: die Detailansicht von Fig. 4 mit Schnittansicht eines Steuerelements der Abwinkelsteuerung in arretiertem Zustand,
- Fig. 5: die Detailansicht wie Fig. 4a, in nicht arretiertem Zustand,
- Fig. 6: das Steuerelement der Abwinkelsteuerung in perspektivischer Einzelan- sicht,
- Fig. 6a: eine Ansicht des Steuerelements von Fig. 6, entlang des Pfeils 93 von Fig. 6,
- Fig. 7: ein Betätigungselement der Abwinkelsteuerung in perspektivischer Einzel- ansicht,
- Fig. 8: ein Reibungselement zur Arretierung der Abwinkelsteuerung in perspekti- vischer Einzelansicht,
- Fig. 9: eine Detailansicht wie in Fig. 4 dargestellt, mit Verlauf von Steuerseilen um eine Trommel,
- Fig. 10: eine Seitenansicht der Trommel von Fig. 9,
- Fig. 10a: einen Schnitt längs der Linie Xa-Xa in Fig. 10,
- Fig. 11: eine Seitenansicht eines Werkzeugeinsatzes für das medizinische Instru- ment von Fig. 1,
- Fig. 12: eine vergrößerte Detailansicht des distalen Endes des Werkzeugs von Fig. 11,
- Fig. 13: Seitenansicht eines Griffteils des in Fig. 1 gezeigten Instruments,
- Fig. 13a: eine Ansicht des Griffteils in Richtung des Pfeils 134 von Fig. 13 betrach- tet,
- Fig. 14: ein vergrößerter abschnittsweiser Schnitt längs der Linie XIV-XIV in Fig. 13, mit eingehängtem Ende des Werkzeugeinsatzes von Fig. 11 im gesperr- ten Zustand,
- Fig. 14a: eine der Fig. 14 entsprechende Darstellung mit freigegebenem Ende des Werkzeugeinsatzes,
- Fig. 15: ein vergrößerter abschnittsweiser Schnitt längs der Linie XV-XV in Fig. 13a, mit eingehängtem Ende des Werkzeugeinsatzes von Fig. 11,
- Fig. 16: eine Sperre in perspektivischer Einzelansicht,
- Fig. 17: eine Detailansicht eines medizinischen Instruments im Bereich des Griffteils zur Veranschaulichung der Rastverbindung,
- Fig. 18: eine Darstellung entsprechend Fig. 17, mit gelöster Rastverbindung,
- Fig. 19: eine Darstellung entsprechend Fig. 17, als Schnitt in der Betrachtungsebe- ne und mit deaktivierter Rastverbindung, und
- Fig. 20: eine Darstellung entsprechend Fig. 19, mit aktivierter Rastverbindung.

Ein in den Figuren dargestelltes medizinisches Instrument ist in seiner Gesamtheit mit der Bezugsziffer 10 bezeichnet.

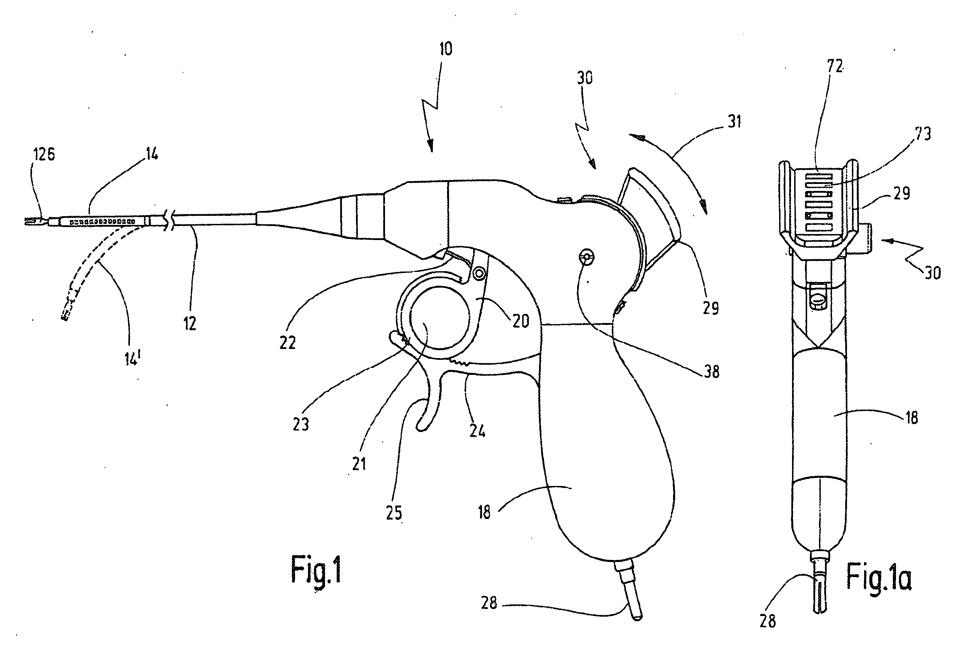

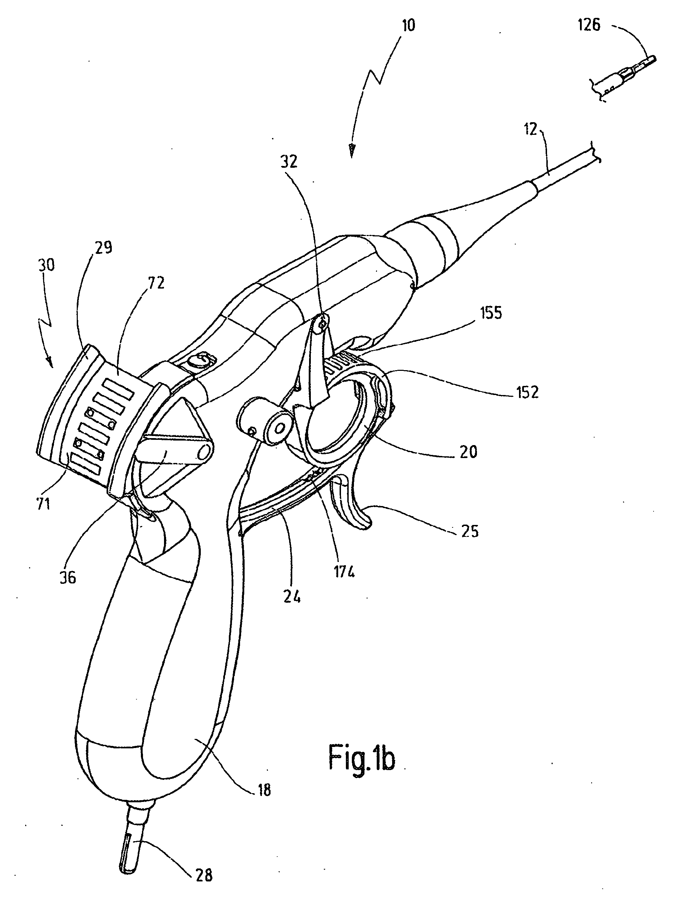

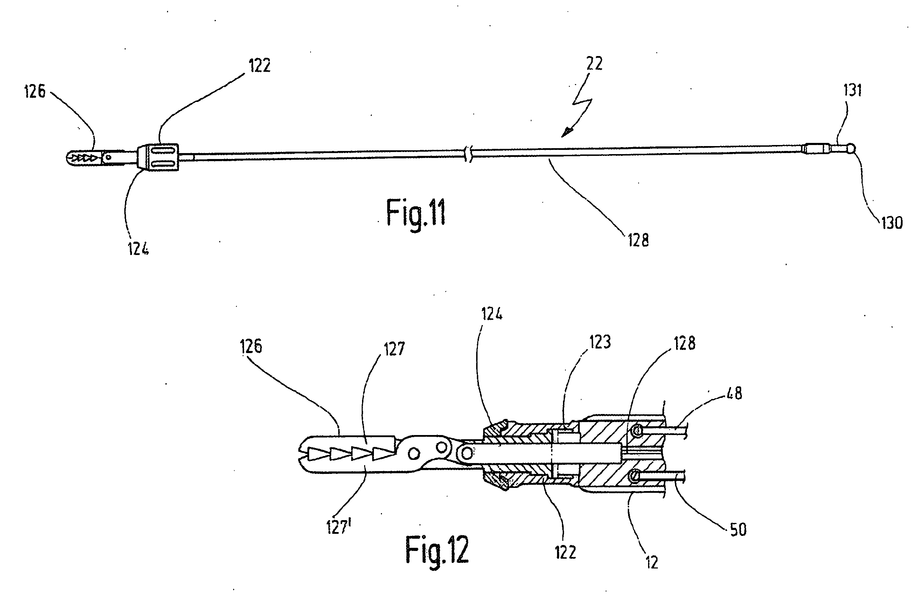

Das in Fig. 1 gezeigte medizinische Instrument 10 weist einen flexiblen Schaft 12 auf, an dessen distalen Ende sich ein abwinkelbarer Bereich 14 befindet. Am Bereich 14 ist distalseitig ein Werkzeug 126 angeordnet. Das Werkzeug 126 stellt ein distales Ende eines in Fig. 11 dargestellten Einsatzes 22 dar. Das proximale Ende des Schaftes 12 ist mit einer Handhabe 18 verbunden.

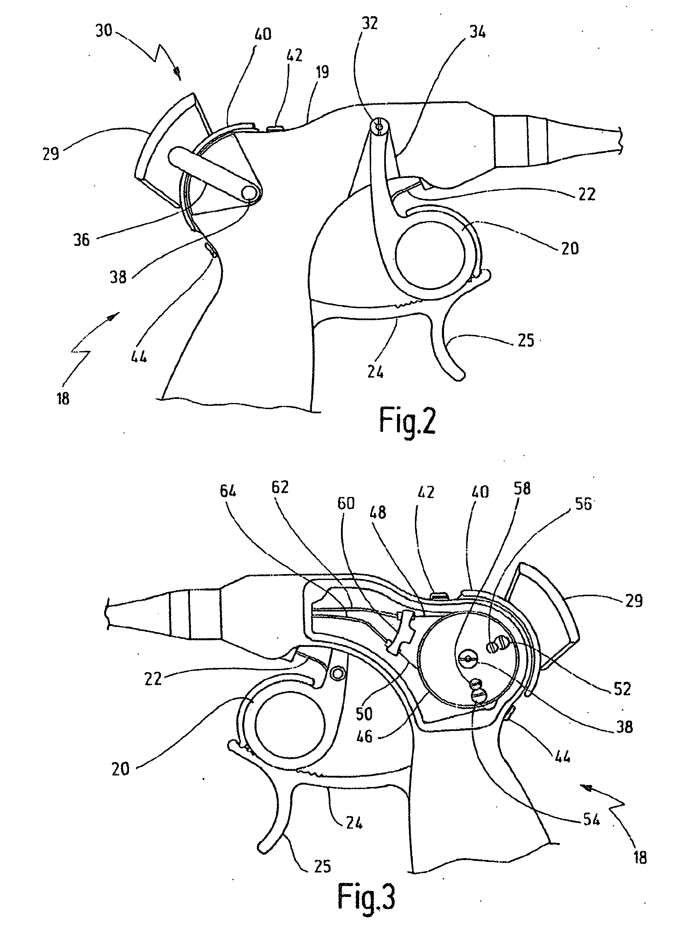

Die Handhabe 18 ihrerseits weist ein bewegliches Griffteil 20 auf. Dieses besitzt eine runde Öffnung 21, die von einem Ringabschnitt 23 umgrenzt ist, durch die vorzugsweise der Zeigefinger des Operateurs geführt werden kann, um eine Bewegung des Griffteils 20, das um die in der Fig. 2 gezeigte Schwenkachse 32 verschwenkbar ist, durchzuführen. Das Griffteil 20 ist mit dem proximalen Ende des Einsatzes 22 verbunden. Durch die Verbindung des Griffteils 20 mit dem Einsatz 22, steht dieser in Wirkverbindung mit dem Werkzeug 126 und dient so zu dessen Betätigung, so z.B. einem Öffnen und Schließen eines Maulteils.

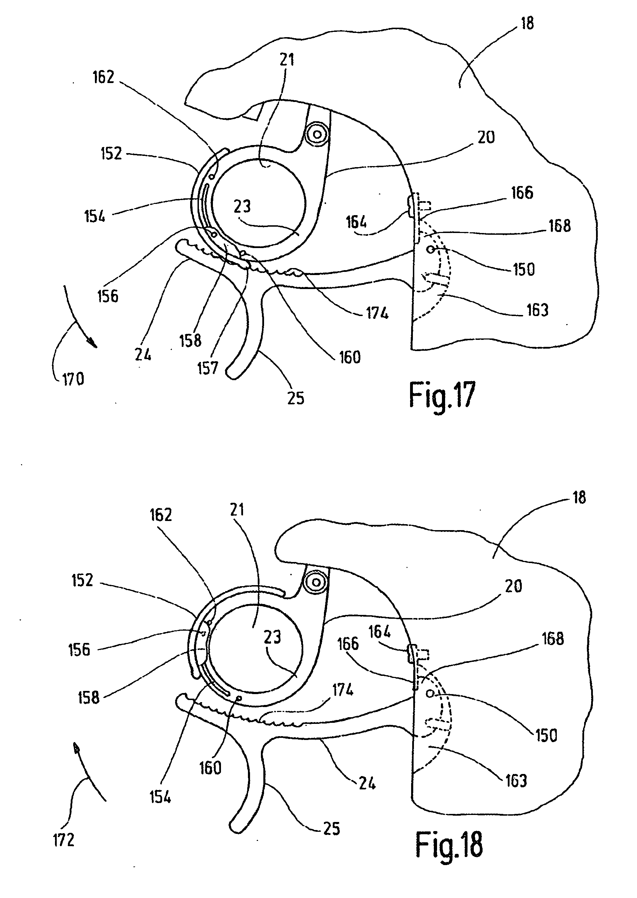

Weiterhin kann das Griffteil 20 in Kontakt mit einer Raste 24 gebracht werden, die eine ungewollte Bewegung des Griffteils 20 in eine distale Richtung verhindern kann. Um eine Lösung der Rastverbindung zu ermöglichen, weist die Raste 24 unter anderem einen bogenförmigen Ansatz 25 auf, der ein Verschwenken der Raste 24 durch den Operateur, vorzugsweise mit dem Mittelfinger, entsprechend der Beschreibung in Zusammenhang mit den Figuren 17ff. ennöglicht.

Des Weiteren ist an der Handhabe 18 ein Steuerelement 29 einer Abwinkelsteuerung 30 angeordnet, durch dessen Bewegung entsprechend der Richtungen des Doppelpfeils 31 um eine Drehachse 38, die senkrecht zur hier dargestellten Achse des Schaftes 12 steht, die Abwinklung des abwinkelbaren Endes 14 des Schaftes 12 gesteuert werden kann. Ein Beispiel für eine Richtung der Abwinklung ist in Fig. 1 durch das abgewinkelte Ende 14' dargestellt.

In Fig. 1a blickt man auf das Steuerelement 29 der Abwinkelsteuerung 30 und ein sich darauf befindliches Betätigungselement 71 in Form eines Drückers 72. Dieser kann durch einen Daumen des Operateurs betätigt werden, wodurch eine Bewegung des Steuerelements 29 ermöglicht wird. Zur besseren Greifbarkeit sind hierfür Rillen 73 auf dem Drücker 72 angebracht.

Das Instrument 10 weist ferner noch einen Stromanschluss 28 auf, der z.B. zur Stromversorgung von optional einsetzbaren Koagulationseinsätzen dienen kann.

Aus Fig. 2 kann man den Bereich der Verschwenkbarkeit des Griffteils 20 um die Schwenkachse 32 im Bereich einer Aussparung 34 erkennen. Das Steuerelement 29 ist über einen Verbindungsarm 36 mit der Drehachse 38 verbunden.

Zwischen einem Gehäuse 19 der Handhabe 18 und dem Steuerelement 29 befindet sich ein Reibungselement 39 in Form eines Reibungsbleches 40, welches durch Schrauben 42 und 44 an der Außenseite der Handhabe 18 befestigt ist. Dieses Reibungsblech 40 dient, wie im Folgenden noch näher beschrieben wird, die Abwinkelsteuerung 30 in einer bestimmten Position zu arretieren.

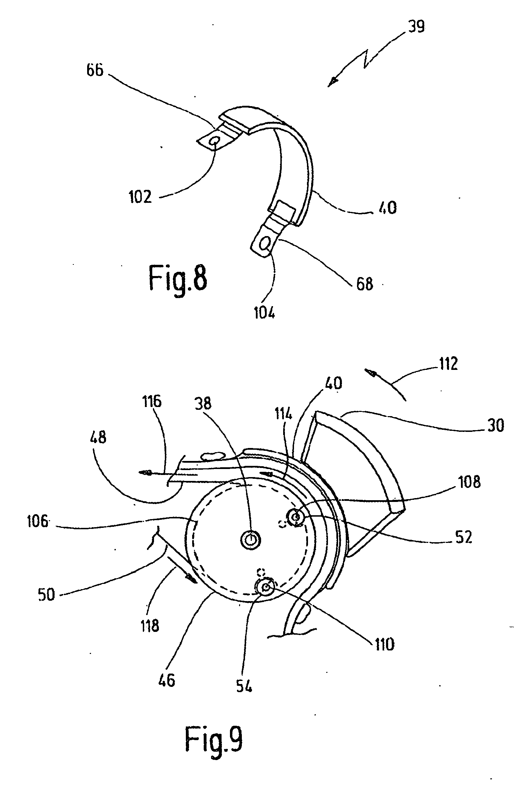

Eine in Fig. 3 erkennbare Trommel 46 ist auf der Drehachse 38 befestigt und steht somit durch den Verbindungsarm 36 mit dem Steuerelement 29 der Abwinkelsteuerung 30 in Wirkverbindung. Eine entsprechende Betätigung des Steuerelements 29 hat somit auch eine direkte Bewegungsübertragung auf die Trommel 46 zur Folge. An der Trommel 46 enden zwei vom abwinkelbaren Ende 14 des Schaftes 12 durch den Schaft 12 verlaufende Steuerseite 48 und 50, die jeweils abseits der Drehachse 38 verlaufen und in diesem Beispiel durch Befestigungsschrauben 52 und 54 in Kombination mit Sicherungsschrauben 56 und 58 an der Trommel 46 befestigt sind. Dazu werden die Steuerseile 48 und 50 von distal her aus Hüllen 62 und 64 austretend durch eine Führung 60 zu der Trommel 46 geleitet. Die Steuerseile 48 und 50 sind die Betätigungselemente für das abwinkelbare Ende 14. Zusammen mit der Trommel 46 und dem Verbindungsarm 36 ergibt sich somit die Wirkverbindung zwischen dem Steuerelement 29 der Abwinkelsteuerung 30 und dem abwinkelbaren Ende 14. Eine detailliertere Funktionsbeschreibung erfolgt später im Zusammenhang mit Fig. 9.

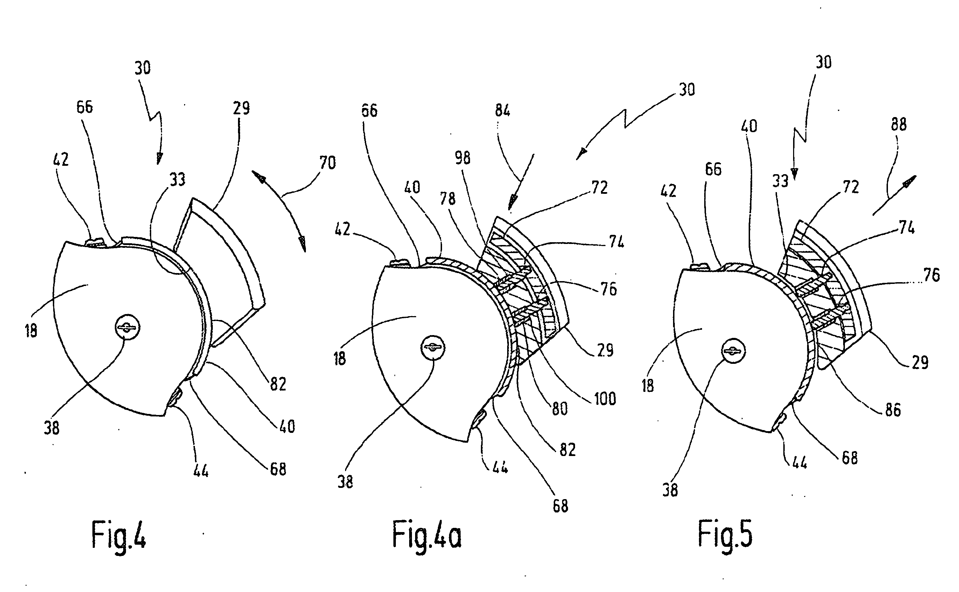

In Zusammenhang mit den Figuren 4 bis 8 wird nunmehr auf die Arretierbarkeit der Abwinkelsteuerung 30 und somit des abwinkelbaren Endes 14 des Schaftes 12 mit Hilfe des Reibungsblechs 40 näher eingegangen.

In Fig. 4 sieht man, dass sich eine Unterseite 33 des Steuerelements 29 in direktem Kontakt mit dem Reibungsblech 40 befindet, welches über abgewinkelte Federbleche 66 und 68 und mittels der Schrauben 42 und 44 an der Handhabe 18 befestigt ist. Das Reibungsblech 40 verläuft somit im Abstand zur Außenseite der Handhabe 18 an der es montiert ist. Eine Bewegung der des Steuerelements 29 um die Drehachse 38 entsprechend der Richtungen des Doppelpfeils 70, wird hierbei durch den reibenden Kontakt zwischen dem Steuerelement 29 und dem Reibungsblech 40 an einer Reibkontaktfläche 82 verhindert, bzw. gebremst.

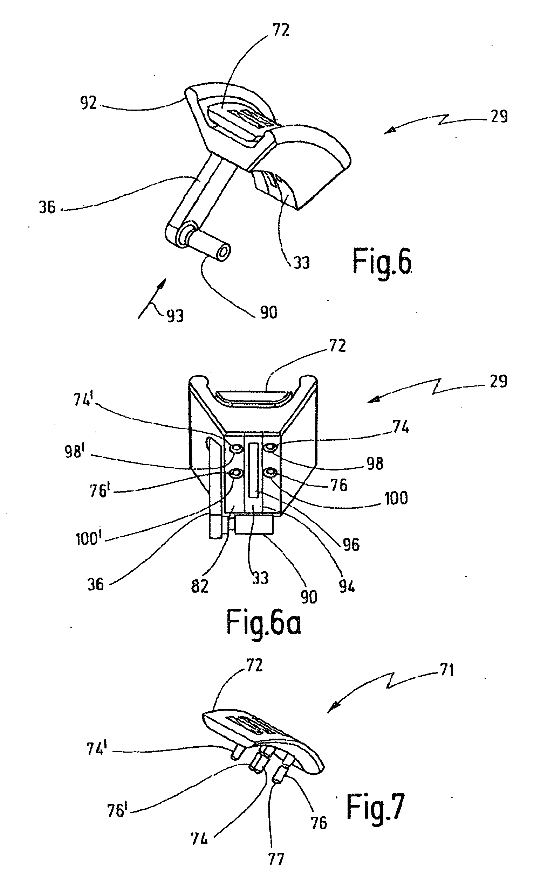

In Fig. 4a ist zu erkennen, dass die Abwinkelsteuerung 30 den Drücker 72 umfasst. Dieser ist, wie auch in Fig. 1b zu erkennen ist, proximalseitig für den Operateur gut zugänglich. Vom Drücker 72 stehen Stifte 74, 74', 76, 76' (siehe auch Fig. 7) vor, die distalseitig durch Hülsen 78, 80 im Körper des Steuerelements 29 geführt und gehalten sind. Die Spitzen der Stifte stehen auf dem Reibungsblech 40 direkt auf und ergeben somit eine Wirkverbindung zwischen dem Drücker 72 und dem Reibungsblech 40. Durch Eindrücken des Drückers 72 entsprechend der Richtung des Pfeils 84, werden die Stifte durch Bohrungen 98, 100 im Körper des Steuerelements 29 axial bewegt, wobei sie das Reibungsblech 40 in Richtung der Handhabe 18 drücken. Das Reibungsblech 40 bewegt sich dadurch von der Unterseite 33 des Steuerelements 29 weg. Die Reibkontaktfläche 82 ist damit gelöst und es ergibt sich ein Spalt 86, wie er in Fig. 5 dargestellt ist. Die für diese Positionsänderung des Reibungsblechs 40 benötigte Flexibilität, wird vor allem durch die Federbleche 66 und 68, aber auch durch Langlochöffnungen 102 und 104, wie sie in Fig. 8 gezeigt sind, ermöglicht.

Die durch Betätigen des Drückers 72 resultierende Stellung, wie sie in Fig. 5 gezeigt ist, erlaubt nun eine reibungsarme Bewegung des Steuerelements 29, wie sie in Fig. 4 anhand des Doppelpfeils 70 gezeigt ist.

Lediglich die Spitzen der vier Stifte 74, 74', 76, 76' stehen auf dem Reibungsblech 40 und gleiten reibungsarm über dessen Oberfläche. Dazu können diese z.B. aus einem reibungsarmen Kunststoffmaterial bestehen. Es kann auch ein metallischer Grundkörper mit dem reibungsarmen Material überzogen sein, oder an einen metallischen Stumpf eine reibungsarme Spitze ausgesetzt sein.

Nimmt nun der Operateur seinen Finger, vorzugsweise den Daumen, wieder vom Drücker 72, so wird aufgrund der Spannung durch die Federbleche 66 und 68 das Reibungsblech 40 wieder gegen die Unterseite 33 des Steuerelements 29 der Abwinkelsteuerung 30 gedrückt, so dass der Spalt 86 verschwindet und wieder die Reibkontaktfläche 82 vorliegt. Entsprechend erfahren die Stifte 74, 74', 76, 76' und damit der Drücker 72 auch wieder eine proximal gerichtete Bewegung entsprechend der Richtung des Pfeils 88. Dadurch wird die Abwinkelsteuerung 30 wieder in ihrer Position arretiert. Dies ist somit im Verschwenkbereich des Steuerelements 29 stufenlos durchzuführen.

Das Steuerelement 29 der Abwinkelsteuerung 30 ist in Fig. 6, 6a und 7 näher dargestellt, wobei man hier deutlich den Drücker 72 und dessen proximale Zugänglichkeit erkennt. Der Drücker 72 ist dabei an einem Fingermuldenteil 92 befestigt, welches über den Verbindungsann 36 und mit einem Achsbolzen 90 an der Drehachse 38 montiert wird.

Fig. 6a zeigt die Unterseite 33, die mit dem Reibungsblech 40 in Kontakt kommt. In diesem Ausführungsbeispiel ist der Drücker 72 mit vier Stiften 74, 74', 76 und 76' ausgestattet, die axial in den Bohrungen 98, 98', 100 und 100' verlaufen und bewegbar sind. Zwischen den beiden Stiftpaaren 74, 76 und 74', 76' ist ein Kunststoffinlet 96 angeordnet, welches durch ein Halteblech 94 am Fingermuldenteil 92 befestigt wird. Dieses Kunststoffinlet 96 dient dazu, die Reibung zwischen dem Steuerelement 29 und dem Reibungsblech 40 zu erhöhen und somit die Arretierung in der gewünschten Position zu stärken.

In Fig. 7 ist der Drücker 72 mit den vier Stiften 74, 74', 76 und 76' gut zu erkennen. Aufgrund ihrer distal abgerundeten Spitzen 77 wird die Reibung bei ihrem Gleiten auf dem Reibungsblech 40 auf ein Minimum reduziert, was die Bedienbarkeit der Abwinkelsteuerung 30 erleichtert.

Das in Fig. 8 gezeigte Ausführungsbeispiel des Reibungsblechs 40 ist an den gegenüberliegenden Enden mit den abgewinkelten Federblechen 66 und 68 verbunden, die beide eine Langlochöffnung 102 bzw. 104 besitzen, wodurch eine Beweglichkeit des Reibungsblechs 40 an der Handhabe 18, entsprechend der vorherigen Beschreibungen, also auf die Handhabe 18 zu bzw. von dieser weg, ermöglicht wird. Die Abwinklungen an den Federblechen 66 und 68 sorgen für die entsprechende Andrückkraft und somit für die entsprechend feste Arretierung zwischen Steuerelement 29 und Handhabe 18 an der das Reibungsblech 40 montiert ist.

Anhand der Fig. 9 bis Fig. 10a soll die Funktionsweise der Abwinkelsteuerung 30 weiter erläutert und die Befestigung der Steuerseile 48 und 50 an der Trommel 46 beschrieben werden.

In Fig. 9 ist der Verlauf der Steuerseile 48 und 50 in der Trommel 46 dargestellt. Letztere umfasst eine umfängliche Rille 106 in der die Steuerseile 48 und 50 geführt werden, um dann in Bohrungen 108 und 109 der Befestigungsschrauben 52 und 54 zu enden. An diesen sind die Steuerseile 48 und 50 dann fest montiert.

Wird nun das Steuerelement 29 in Richtung des Pfeils 112 bewegt, so erfährt die Trommel 46, durch die zuvor beschriebene Wirkverbindung über den Verbindungsarm 36, eine Drehbewegung um die Drehachse 38, wie sie durch die Richtung des Pfeils 114 angegeben ist. Für die an der Trommel 46 befestigten Steuerseile 48 und 50 bedeutet dies, dass sie ebenfalls eine Bewegung erfahren und zwar wird das Steuerseil 48 in Richtung des Pfeils 116 in den Schaft 12 hineingeschoben und das Steuerseil 50 entsprechend in Richtung des Pfeils 118 aus dem Schaft herausgezogen. Aufgrund der bereits erwähnten Wirkverbindung zwischen den Steuerseilen 48 und 50 mit dem abwinkelbaren Ende 14, wird dieses in seiner Winkelstellung folglich verändert. Dies resultiert in einem Abwinkeln entsprechend der in Fig. 1 durch das abwinkelbare Ende 14' dargestellten Fonn.

Die entgegengesetzte Bewegung führt wieder zu einem Strecken des Schaftes 12 bzw. einem Abwinkeln, aus der Sicht von Fig. 1, nach oben. Durch Freigabe des Drückers 72 kann die Position bzw. Abwinklung des abwinkelbaren Endes 14 in jeder beliebigen Stellung arretiert werden.

Die Anordnung der Trommel und die des Steuerelements um 90° verdreht, würde anstatt der in Fig. 1 dargestellten Abwinkelebene "oben-unten" eine um die Schaftachse um 90° verdrehte Abwinkelebene "links-rechts" ergeben. Man kann die Steuerseile auch vertauscht anbringen, dann führt z.B. ein Verschieben des Steuerelements "nach vorne" anstatt zu einer Abwinklung nach "unten" zu einer Abwinklung nach "oben".

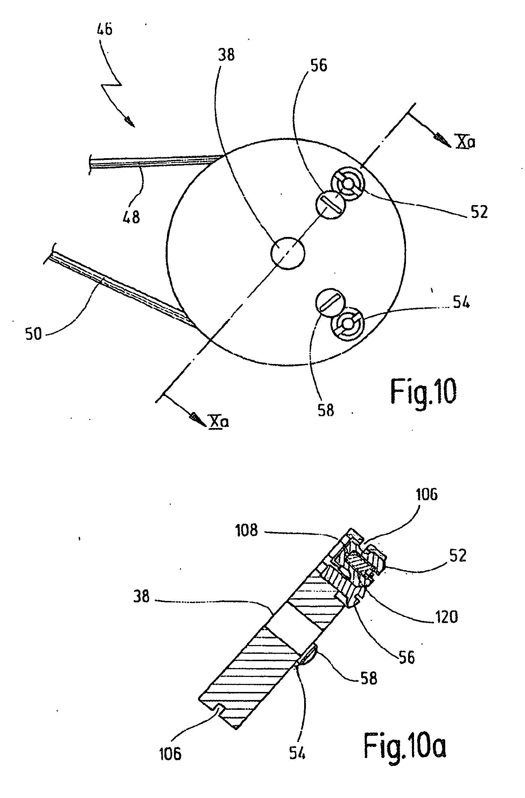

In Fig. 10a erkennt man die Rille 106. Des Weiteren sieht man die Bohrung 108 der Befestigungsschraube 52. Durch diese wird in dem hier erwähnten Beispiel das Steuerseil 48 in die Befestigungsschraube 52 eingeführt und mittels einer Fixierschraube 120 in dieser Befestigungsschraube fest montiert. Entsprechendes gilt für die hier nicht im Querschnitt gezeigte Befestigungsschraube 54 und das Steuerseil 50. Anschließend kann dann die Länge der Steuerseile 48 und 50 durch individuelle Drehung der Schrauben 52 und 54 eingestellt werden. In einem Ausführungsbeispiel haben diese dazu unterschiedlich verlaufende Gewinde, so dass Befestigungsschraube 52 ein Rechts- und Befestigungsschraube 54 ein Linksgewinde aufweist. Nach erfolgter Einstellung der Steuerseile 48 und 50 erfolgt dann die Fixierung der Befestigungsschrauben 52 und 54 mittels der Sicherungsschrauben 56 und 58. Diese verhindern eine eigenständige Drehung der Befestigungsschrauben 52 und 54 und somit ein ungewolltes Verstellen der Steuerseile 48 und 50.

In den folgenden Figuren 11 bis 16 wird die Ausgestaltung und die Montage des Einsatzes 22 beschrieben.

Der in Fig. 11 gezeigte Einsatz 22 weist distal ein Werkzeug 126, hier zwei spreizbare Maulteile 127, 127' auf, welches über ein stabförmiges flexibles Betätigungselement 128 mit einem Verbindungsstück 130 in Wirkverbindung steht. Proximal hinter dem Werkzeug 126 ist eine Haube 124 und ein Schraubverschluss 122 angebracht, die beide zur Befestigung des Einsatzes 22 an einem flexiblen Schaft, wie z.B. an den flexiblen Schaft 12 aus Fig. 1, dienen. Das proximale Ende des Einsatzes 22 weist, wie zuvor bereits erwähnt, das Verbindungsstück 130 auf, welches bspw. zur Befestigung an dem Griffteil 20 des medizinischen Instruments 10 dient. Dazu ist dieses in diesem Ausführungsbeispiel das Ende kugelförmig ausgestaltet und proximal hinter einem durchmessergeringerem Abschnitt 131 am Einsatz 22 angeordnet.

Fig. 12 zeigt die Befestigung des distalen Endes des Einsatzes 22 am distalen Ende des Schafts 12. Dabei ist die proximal hinter dem Werkzeug 126 befindliche Haube 124 fest mit dem Einsatz 22 verbunden. Dadurch wird ein Verrutschen des Schraubverschlusses 122 in eine distale Richtung verhindert. Dieser Schraubverschluss 122 wird seinerseits dann auf ein Außengewinde 123 am distalen Ende des Schaftes 12 aufgeschraubt. Das Kraftübertragungselement 128 wird dazu zuvor von distal in den Schaft 12 eingeführt. Durch diese Befestigung ist das distale Ende des Einsatzes 22 lagefixiert. Ein Abwinkeln des abwinkelbaren Endes 14 verursacht dann nicht mehr ein Ausschieben des Einsatzes 22 aus dem distalen Ende des Schafts 12.

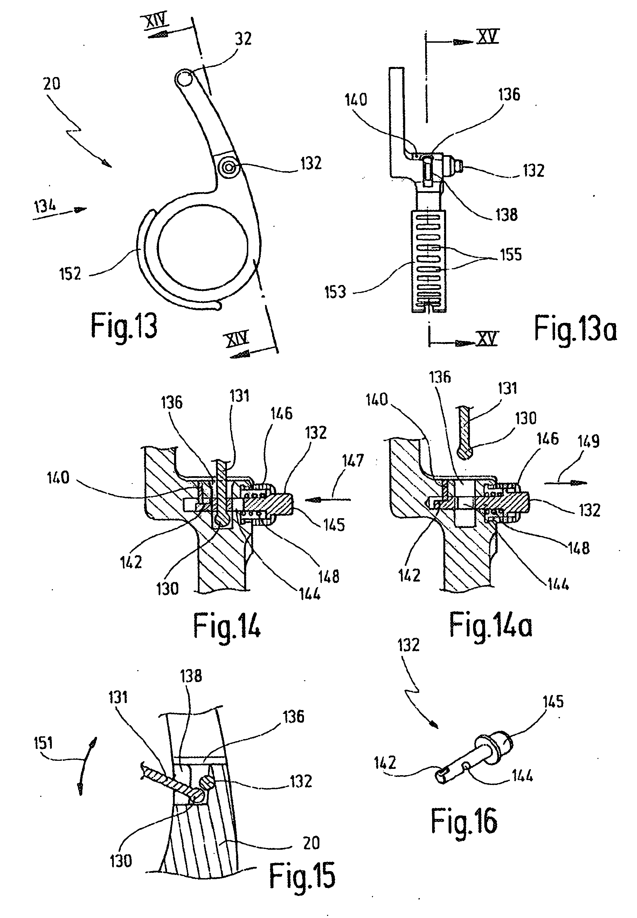

Die Fig. 13 bis 16 zeigen das Griffteil 20, dessen Schwenkachse 32 und einer Sperre 132 zum lösbaren Verbinden mit dem proximalen Ende des Einsatzes 22. In Fig. 13a erkennt man eine in Richtung der Schwenkachse 32 mündende Öffnung 136 durch die das kugelförmige Ende des Verbindungsstücks 130 eingeführt wird. Der distal folgende durchmessergeringe Abschnitt 131 am Einsatz 22 kann über eine Nut 138 seitlich aus dem Inneren des Griffteils 20 herausgeführt werden (siehe Fig. 15). Um das Ende des Einsatzes 22 einzuführen, muss eine Sperre 132 gedrückt werden, so dass das Verbindungsstück 130 diese passieren kann. Dies lässt sich an den Figuren 14 und 14a erkennen.

Die in Fig. 16 ersichtliche Sperre 132 wird durch eine Halterung 146 am Griffteil 20 gehalten. Durch eine Feder 148 wird diese ferner gegen den Rand dieser Halterung 146 gedrückt. Die in Fig. 14 gezeigte Position stellt somit die Grundstellung der Sperre 132 dar. Man sieht hierbei, wie das Verbindungsstück 130 aufgrund seines hier kugelförmigen Endes durch die Sperre 132 blockiert wird und somit nicht mit Bezug auf die Darstellung nach oben durch die Öffnung 136 gelangen kann. Betätigt man nun die Sperre 132 entgegen der Richtung, in der sie durch die Feder 148 gedrückt wird, also in Richtung des Pfeiles 147, so bewegt sich eine Aussparung 144 an der Sperre 132, die sich in der Darstellung von Fig. 14 rechts des Verbindungsstückes 130 befindet, in eine zentrale Position der Öffnung 136, wie sie bspw. in Fig. 14a gezeigt ist. Dieses Eindrücken kann über einen Knopf 145 durchgeführt werden, der seitlich über die Halterung 146 hinaus zur Außenseite ragt. Diese Aussparung 144 gibt dem kugelförmigen Verbindungsstück 130 gerade genügend Platz, um an dieser Sperre 132 vorbeizugelangen. Dadurch kann das Verbindungsstück 130 durch die Öffnung 136 hindurch aus der Halterung in dem Grillteil 20 entfernt werden. Gibt man die Sperre 132 wieder frei, so bewegt sich diese entsprechend der Richtung des Pfeils 149 aus Fig. 14a wieder nach außen. Ursache hierfür ist wiederum die Feder 148. Gleichzeitig verschiebt sich dabei dann auch die Aussparung 144.

Will man das Verbindungsstück 130 nun wieder in die Halterung des Griffteils 20 einsetzen, so bedarf es wieder einem Eindrücken der Sperre 132 in Richtung des Pfeils 147 aus Fig. 14, so dass die Aussparung 144 wieder in die zentrale Lage, wie sie in Fig. 14a gezeigt ist, zu liegen kommt. Auf diese Weise kann man nun das kugelförmige Ende wieder an der Sperre 132 vorbeiführen und das Verbindungsstück 130 durch die Öffnung 136 am Griffteil 20 befestigen.

In Fig. 15 sieht man mit einem Verbindungsstück 130 veranschaulicht, wie sich dieses unterhalb der Sperre 132 befindet. Eine Bewegung nach oben ist nicht möglich. Der durchmessergeringe Abschnitt 131 am proximalen Ende des Einsatzes 22 passt durch die Nut 138 hindurch und somit ist eine Bewegbarkeit entsprechend des Doppelpfeils 151 ermöglicht wird. Diese Bewegungsfreiheit wird bei der Bewegung des Griffteils 20 benötigt.

Um ein Verdrehen der um die Längsachse drehbaren Sperre 132 und damit auch ein Verdrehen der Aussparung 144 zu verhindern, ist am distalen Ende der Sperre 132 eine axiale Nut 142 eingearbeitet. Diese dient auch als Anschlag für die Verschiebebewegung. Dies ist in den Figuren 14 und 14a, sowie auch in der perspektivischen Darstellung der Fig. 16 gezeigt. In dieser Nut 142 endet nun bei der Befestigung im Griffteil 20 ein Stift 140, der zwar ein unerwünschtes Drehen um die Längsachse der Sperre 132 verhindert, eine axiale Beweglichkeit der Sperre 132 in Richtung der Pfeile 147 und 149 jedoch erlaubt.

In den Figuren 17 bis 20 ist die Rastverbindung, die sich durch die Raste 24 auf das Griffteil 20 ergibt, näher erläutert.

Die Raste 24 ist an einer Schwenkachse 150 verschwenkbar an der Handhabe 18 in einer Aussparung 163 angebracht. Durch Kontakt mit dem Griffteil 20 kann diese Raste 24 in diesem Ausführungsbeispiel die Bewegung des Griffteils 20 in die distale Richtung unterbinden. Dazu wird die Raste 24 durch die Vorspannung aufgrund eines Federblechs 166 in Richtung des Griffteils 20 gedrückt.

Die Raste 24 weist dazu an der dem Griffteil 20 zugewandten Seite Rastzähne 174 auf, die mit einem Raststift 160 am Griffteil 20 in Eingriff kommen. Die Neigung der Flanken der Rastzähne 174 in Richtung Handhabe 18, erlauben eine Bewegung des Griffteils 20 in Richtung der Handhabe 18, sperren diese jedoch in der entgegengesetzten Richtung.

Will man die Rastverbindung kurzzeitig lösen, so verschwenkt man die Raste 24 entsprechend der Richtung des Pfeils 170, vorzugsweise durch Betätigung am bogenförmigen Ansatz 25, was zu einer Endstellung, wie sie in Fig. 18 gezeigt ist, führt. Aufgrund der Vorspannung wird die Raste 24 bei Loslassen dieser entsprechend der Richtung des Pfeils 172 wieder zurück an den Griffteil 20 gebracht.

Um für einen bestimmten Zeitraum die Rastverbindung zu deaktivieren, ist am Griffteil 20 eine Rastsperre 152 angebracht.

Die Rastsperre 152 ist als ein gebogenes Element, im dargestellten Ausführungsbeispiel als ein gebogener Streifen 153 ausgebildet (siehe auch Fig. 13a) dessen Krümmung der Krümmung der Außenseite des Ringabschnittes 23 des Griffteils 20 angepasst ist.

Aussparungen bzw. Austanzungen 155 im Streifen 153 erhöhen die Griffigkeit.

Diese Rastsperre 152 kann, wie aus Fig. 17 ersichtlich, zwischen Griffteil 20 und Raste 24 gebracht werden. In diesem Fall ist die Rastverbindung deaktiviert und das Griffteil 20 ist frei in beide Richtungen bewegbar. Dazu weist die Rastsperre 152 eine abgerundete Backe 157 auf, die in beiden Richtungen über die Zähne 174 laufen kann. Dies entspricht einer zweiten Stellung der Rastsperre 152. Um die Rastverbindung nun wieder zu aktivieren, kann die Rastsperre 152 in Richtung eines Raststifts 162 geschoben werden. Dies entspricht einer ersten Stellung der Rastsperre 152. Auf beiden Seiten des Streifens 153 ist eine Abdeckung 158 vorhanden. Diese deckt einen in der Rastsperre quer verlaufenden Führungsstift 156 ab, der in beidseitigen Führungsnuten 154 im Ringabschnitt 23 läuft. Die Abdeckungen 158 selbst können über hier nicht näher dargestellte Stifte an der Rastsperre 152 befestigt werden. Dementsprechend durchläuft die Rastsperre 152 eine kreisförmige Bewegung, wie sie durch die Form des Ringabschnitts 23 des Griffteils 20 vorgegeben ist und endet somit in einer Position, wie sie in Fig. 18 gezeigt ist. Dadurch liegt dann ein zuvor durch die Rastsperre 152 blockierter Raststift 160 frei und kann mit den Zähnen 174 der Raste 24 in Eingriff treten.

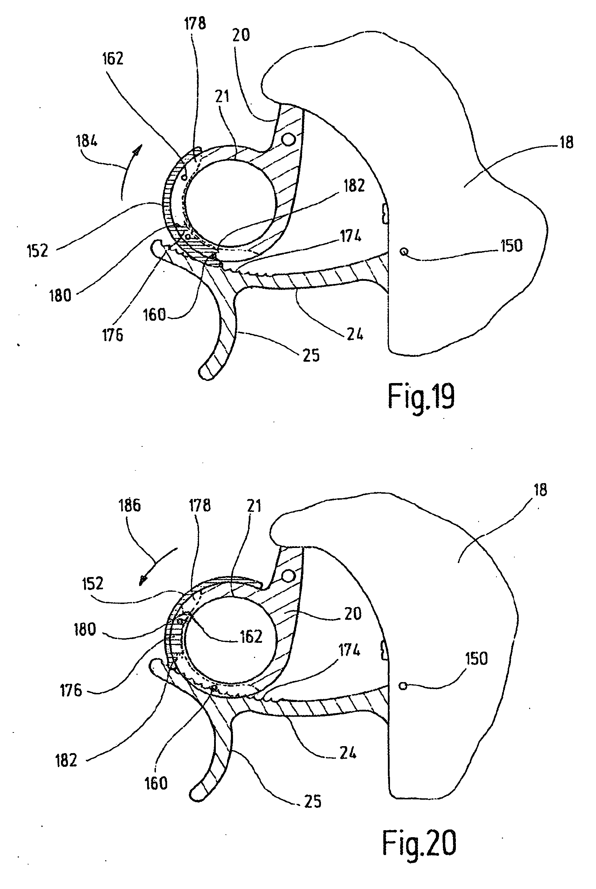

Wie in den Figuren 19 und 20 gezeigt ist, ist umfänglich im Griffteil 20 eine äußere Nut 178 eingeschnitten. In dieser ist eine in radialer Richtung des Ringabschnittes 23 vorspringende Stufe 176 der Rastsperre 152 bewegbar, die mittig an der Rastsperre 152 angeordnet ist. An jeweils einem Ende dieser Stufe 176 befinden sich Federclips 180 bzw. 182. Diese können entsprechend der Position der Rastsperre 152 in den Raststift 160 bzw. 162 einrasten und damit ein einfaches Hin- und Herrutschen der Rastsperre 152 verhindern. Diese wird somit in den jeweiligen Stellungen gehalten.

Fig. 19 zeigt in diesem Zusammenhang die zweite Stellung der Rastsperre 152, in der die Rastverbindung deaktiviert ist. Der Federclip 182 der Stufe 176 an der Rastsperre 152 ist in den Raststift 160 eingerastet, und blockiert somit den Kontakt zwischen den Rastzähnen 174 und dem Raststift 160. Eine Bewegung der Rastsperre 152 in Richtung des Pfeils 184 würde schließlich in der ersten Stellung enden, wie sie in Fig. 20 gezeigt ist. Dabei ist der Federclip 180, der sich an der Stufe 176 befindet, in den Raststift 162 eingerastet, und damit ist die Rastsperre 152 in dieser Position fixiert. Der Raststift 160 liegt somit frei und kann in die Zähne 174 der Raste 24 einhaken.

Dahingegen ist eine proximale Bewegung des Griffteils 20, die z.B. zu einem Schließen der Maulteile 127, 127' führen würde, über die Raste 24 weiter möglich.

Eine Deaktivierung der Rastverbindung kann nun wieder durch Bewegung der Sperre 152 analog zu dem zuvor gesagten entsprechend der Richtung des Pfeils 186, vorzugsweise nach vorherigem Absenken der Raste 24, gemäß der Beschreibung zu den Figuren 17 und 18, erfolgen.

Die Handhabungsperson kann das Instrument 10, wie in Fig. 1a ersichtlich, über die Handhabe 18 ergreifen. Mit dem Daumen kann der Drücker 72 gedrückt werden und dann kann das Steuerelement 29 verschoben werden. Dies bewirkt ein entsprechendes Abwinkeln des abwinkelbaren Endes 14 des Schafts. Ein Freigeben des Drückers 72 arretiert das abwinkelbaren Ende 14 in der entsprechenden Stellung.

Ein Bewegen des Griffteils 20, z.B. mit dem eingeschobenen Zeigerfinger, erlaubt das Öffnen und Schließen der Maulteile 127, 127' über den Einsatz 22 in jeder beliebigen Abwinkelstellung des abwinkelbaren Endes 14 des Schaftes 12.

Bei deaktivierter Rastfunktion ist die Bewegung des Griffteils 20 in beiden Schwenkrichtungen möglich.

Bei aktivierter Rastfunktion kann diese kurzzeitig durch Verschwenken der Raste 24 mit dem Mittelfinger über den bogenförmigen Ansatz 25 bewerkstelligt werden.

Die Handhabungsperson kann somit auf sehr ergonomische Art und Weise das medizinische Instrument 10 einfach und sicher bedienen.

## Patentansprüche

1. Medizinisches Instrument, mit einem Schaft (12), der proximal mit einer Handhabe (18) verbunden ist, mit einem längs des Schaftes. (12) geführten Einsatz (22), der proximalseitig mit einem beweglichen Griffteil (20) der Handhabe (18) verbunden ist und distal ein Werkzeug (126) aufweist, das durch Verschwenken des Griffteiles (20) betätigbar ist, und mit einer Raste (24), die mit dem beweglichen Griffteil (20) in rastenden Eingriff bringbar ist, **dadurch gekennzeichnet, dass** eine Rastsperre (152) vorhanden ist, die zwischen Raste (24) und Griffteil (20) bringbar ist, so dass die Rastfunktion durch die Rastsperre (152) aufhebbar ist.

2. Medizinisches Instrument nach Anspruch 1, **dadurch gekennzeichnet, dass** die Rastsperre (152) aus einer ersten Stellung, in der die Rastfunktion unbeeinflusst ist in eine zweite Stellung bringbar ist, in der die Rastfunktion aufgehoben ist.

3. Medizinisches Instrument nach Anspruch 2, **dadurch gekennzeichnet, dass** die Rastsperre (152) in der ersten Stellung haltbar ist.

4. Medizinisches Instrument nach Anspruch 2 oder 3, **dadurch gekennzeichnet, dass** die Rastsperre (152) in der zweiten Stellung haltbar ist.

5. Medizinisches Instrument nach einem der Anspruche 1 bis 4, **dadurch gekennzeichnet, dass** die Rastsperre (152) in einer führbar ist.

6. Medizinisches Instrument nach Anspruch 5, **dadurch gekennzeichnet, dass** die Führung zumindest eine Nut (154, 178) aufweist, in die ein vorspringendes Element der Rastsperre (152) eingreift.

7. Medizinisches Instrument nach einem der Ansprüche 1 bis 6, **dadurch gekennzeichnet, dass** die Rastsperre (152), in dem Bereich, der mit Zähnen (174) der Raste (24) in Kontakt tritt, abgerundet ausgebildet ist.

8. Medizinisches Instrument nach einem der Ansprüche 1 bis 7, **dadurch gekennzeichnet, dass** die Rastsperre (152) am beweglichen Griffteil (20) angebracht ist.

9. Medizinisches Instrument nach Anspruch 8, **dadurch gekennzeichnet, dass** die Rastsperre (152) am Griffteil (20) verschiebbar angebracht ist, und dort zwischen der ersten und der zweiten Stellung verschiebbar ist.

10. Medizinisches Instrument nach Anspruch 8 oder 9, **dadurch gekennzeichnet, dass** das bewegliche Griffteil (20) eine Fingeröffnung (21) aufweist, an deren Ringabschnitt (23) die Rastsperre (152) angebracht ist.

11. Medizinisches Instrument nach Anspruch 10, **dadurch gekennzeichnet, dass** die Rastsperre (152) als gebogenes Element (153) ausgebildet ist, dessen Krümmung dem Ringabschnitt (23) des Griffteiles (20) angepasst ist.

12. Medizinisches Instrument nach Anspruch 11, **dadurch gekennzeichnet, dass** das gebogene Element (153) Merkmale aufweist, die dessen Griffigkeit erhöhen.

13. Medizinisches Instrument nach Anspruch 12, **dadurch gekennzeichnet, dass** die Merkmale zur Erhöhung der Griffigkeit ausgewählt sind aus Erhebungen, Riffelungen, Aussparungen (155), Ausstanzungen, Mulden.

14. Medizinisches Instrument nach einem der Ansprüche 8 bis 13, **dadurch gekennzeichnet, dass** von der Rastsperre (152) Führungsstifte (156) vorspringen, die in jeweils eine Nut (154) an den Seiten des Ringabschnittes (23) eingreifen.

15. Medizinisches Instrument nach einem der Ansprüche 8 bis 13, **dadurch gekennzeichnet, dass** die Rastsperre (152) seitliche Abdeckungen (158) aufweist, die den Ringabschnitt (23) des beweglichen Griffteiles (20) seitlich übergreifen.

16. Medizinisches Instrument nach einem der Ansprüche 8 bis 14, **dadurch gekennzeichnet, dass** sich die Rastsperre (152) entlang der Außenseite des Ringabschnittes (23) erstreckt.

17. Medizinisches Instrument nach einem der Ansprüche 2 bis 16, **dadurch gekennzeichnet, dass** an der Rastsperre (152) Clip-Elemente (180, 182) angeordnet sind, die in der ersten bzw. der zweiten Stellung mit Haltestiften (160, 162) in Verbindung treten.

18. Medizinisches Instrument nach Anspruch 17, **dadurch gekennzeichnet, dass** in der zweiten Stellung das Clip-Element (182) mit dem Raststift (160) in haltende Verbindung tritt, der mit der Raste (24) zusammenwirkt.

19. Medizinisches Instrument nach Anspruch 17 oder 18, **dadurch gekennzeichnet, dass** die Clip-Elemente (180, 182) an einer Stufe (176) angeordnet sind, die sich in eine umfängliche Nut (178) hineinerstrecken, die am beweglichen Griffteil (20) ausgespart ist.

20. Medizinisches Instrument nach einem der Ansprüche 1 bis 19, **dadurch gekennzeichnet, dass** die Raste (24) als in Richtung des beweglichen Griffteils (20) vorgespannter verschwenkbarer Hebel ausgebildet ist, der an der Handhabe (18) angelenkt ist.

21. Medizinisches Instrument nach einem der Ansprüche 1 bis 20, **dadurch gekennzeichnet, dass** die Raste (24) einen Ansatz (25) aufweist, über den die Raste (24) von dem beweglichen Griffteil (20) abhebbar ist.

22. Medizinisches Instrument nach Anspruch 21, **dadurch gekennzeichnet, dass** der Ansatz (25) bogenförmig ausgebildet ist, in den ein Finger einer Hand, die die Handhabe (18) ergriffen hat, legbar ist.

## Claims

1. Medical instrument with a shaft (12) which is connected at the proximal end to a handle (18), with an insert (22) which is guided along the shaft (12) and which is connected at the proximal end to a movable grip part (20) of the handle (18) and comprises a tool (126) at its distal end which can be actuated by pivoting of the grip part (20), and with a lock (24) which can be brought into locking engagement with the movable grip part (20), **characterized in that** a lock detent (152) is provided which can be brought between lock (24) and grip part (20), such that the lock function can be cancelled by the lock detent (152).

2. Medical instrument according to Claim 1, **characterized in that** the lock detent (152) can be brought from a first position, in which the lock function is unaffected, to a second position, in which the lock function is cancelled.

3. Medical instrument according to Claim 2, **characterized in that** the lock detent (152) can be held in the first position.

4. Medical instrument according to Claim 2 or 3, **characterized in that** the lock detent (152) can be held in the second position.

5. Medical instrument according to one of Claims 1 to 4, **characterized in that** the lock detent (152) can be guided in a guide.

6. Medical instrument according to Claim 5, **characterized in that** the guide comprises at least one groove (154, 178) in which a projecting element of the lock detent (152) engages.

7. Medical instrument according to one of Claims 1 to 6, **characterized in that** the lock detent (152) is rounded in the area that comes into contact with teeth (174) of the lock (24).

8. Medical instrument according to one of Claims 1 to 7, **characterized in that** the lock detent (152) is mounted on the movable grip part (20).

9. Medical instrument according to Claim 8, **characterized in that** the lock detent (152) is mounted displaceably on the grip part (20) and can be displaced there between the first and second positions.

10. Medical instrument according to Claim 8 or 9, **characterized in that** the movable grip part (20) comprises a finger opening (21) on whose ring section (23) the lock detent (152) is mounted.

11. Medical instrument according to Claim 10, **characterized in that** the lock detent (152) is designed as a curved element (153) whose curvature is adapted to the ring section (23) of the grip part (20).

12. Medical instrument according to Claim 11, **characterized in that** the curved element (153) comprises features that increase its grip.

13. Medical instrument according to Claim 12, **characterized in that** the features for increasing the grip are chosen from elevations, flutings, recesses (155), punches, hollows.

14. Medical instrument according to one of Claims 8 to 13, **characterized in that** guide pins (156) project from the lock detent (152) that engage in a respective groove (154) on the sides of the ring section (23).

15. Medical instrument according to one of Claims 8 to 13, **characterized in that** the lock detent (152) comprises lateral covers (158) that engage laterally over the ring section (23) of the movable grip part (20).

16. Medical instrument according to one of Claims 8 to 14, **characterized in that** the lock detent (152) extends along the outer face of the ring section (23).

17. Medical instrument according to one of Claims 2 to 16, **characterized in that** clip elements (180, 182) are arranged on the lock detent (152) and enter into connection with retaining pins (160, 162) in the first and second positions, respectively.

18. Medical instrument according to Claim 17, **characterized in that**, in the second position, the clip element (182) enters into a retaining connection with the locking pin (160) that engages with the lock (24).

19. Medical instrument according to Claim 17 or 18, **characterized in that** the clip elements (180, 182) are arranged on a step (176) extending into a circumferential groove (178) that is cut out on the movable grip part (20).

20. Medical instrument according to one of Claims 1 to 19, **characterized in that** the lock (24) is designed as a pivotable lever which is pretensioned in the direction of the movable grip part (20) and which is articulated on the handle (18).

21. Medical instrument according to one of Claims 1 to 20, **characterized in that** the lock (24) comprises an attachment (25) via which the lock (24) can be withdrawn from the movable grip part (20).

22. Medical instrument according to Claim 21, **characterized in that** the attachment (25) is arc-shaped and can receive a finger of the hand that has taken hold of the handle (18).

## Revendications

1. Instrument médical comprenant un fût (12) relié à une poignée (18) dans la région proximale ; une pièce intégrée (22) qui est guidée le long dudit fût (12), est reliée, côté proximal, à une partie mobile de préhension (20) de ladite poignée (18) et comporte, dans la région distale, un outil (126) pouvant être actionné par pivotement de ladite partie de préhension (20) ; et un organe (24) à crans d'arrêt pouvant être mis en prise d'encliquetage avec ladite partie mobile de préhension (20), **caractérisé par** la présence d'un bloqueur de crantage (152) pouvant être interposé entre ledit organe (24) à crans d'arrêt et ladite partie de préhension (20), de telle sorte que la fonction d'encliquetage puisse être neutralisée par ledit bloqueur de crantage (152).

2. Instrument médical selon la revendication 1, **caractérisé par le fait que** le bloqueur de crantage (152) peut être déplacé d'une première position, dans laquelle la fonction d'encliquetage n'est pas influencée, à une seconde position dans laquelle ladite fonction d'encliquetage est neutralisée.

3. Instrument médical selon la revendication 2, **caractérisé par le fait que** le bloqueur de crantage (152) peut être retenu dans la première position.

4. Instrument médical selon la revendication 2 ou 3, **caractérisé par le fait que** le bloqueur de crantage (152) peut être retenu dans la seconde position.

5. Instrument médical selon l'une des revendications 1 à 4, **caractérisé par le fait que** le bloqueur de crantage (152) peut être guidé dans un guide.

6. Instrument médical selon la revendication 5, **caractérisé par le fait que** le guide présente au moins une rainure (154, 178) dans laquelle pénètre un élément saillant du bloqueur de crantage (152).

7. Instrument médical selon l'une des revendications 1 à 6, **caractérisé par le fait que** le bloqueur de crantage (152) est de réalisation arrondie dans la région entrant en contact avec des dents (174) de l'organe (24) à crans d'arrêt.

8. Instrument médical selon l'une des revendications 1 à 7, **caractérisé par le fait que** le bloqueur de crantage (152) est implanté sur la partie mobile de préhension (20).

9. Instrument médical selon la revendication 8, **caractérisé par le fait que** le bloqueur de crantage (152) est implanté de manière coulissante sur la partie de préhension (20), sur laquelle il peut coulisser entre les première et seconde positions.

10. Instrument médical selon la revendication 8 ou 9, **caractérisé par le fait que** la partie mobile de préhension (20) présente un orifice (21) d'engagement d'un doigt, sur la région annulaire (23) duquel le bloqueur de crantage (152) est implanté.

11. Instrument médical selon la revendication 10, **caractérisé par le fait que** le bloqueur de crantage (152) est réalisé sous la forme d'un élément arqué (153) dont la courbure est adaptée à la région annulaire (23) de la partie de préhension (20).

12. Instrument médical selon la revendication 11, **caractérisé par le fait que** l'élément arqué (153) offre des attributs qui augmentent sa maniabilité.

13. Instrument médical selon la revendication 12, **caractérisé par le fait que** les attributs augmentant la maniabilité sont sélectionnés parmi des protubérances, des striures, des évidements (155), des découpes matricées ou des augettes.

14. Instrument médical selon l'une des revendications 8 à 13, **caractérisé par le fait que** des tétons de guidage (156), faisant saillie au-delà du bloqueur de crantage (152), pénètrent respectivement dans une rainure (154) sur les côtés de la région annulaire (23).

15. Instrument médical selon l'une des revendications 8 à 13, **caractérisé par le fait que** le bloqueur de crantage (152) est doté d'éléments latéraux de recouvrement (158) coiffant, dans le sens latéral, la région annulaire (23) de la partie mobile de préhension (20).

16. Instrument médical selon l'une des revendications 8 à 14, **caractérisé par le fait que** le bloqueur de crantage (152) s'étend le long de la face extérieure de la région annulaire (23).

17. Instrument médical selon l'une des revendications 2 à 16, **caractérisé par le fait que** des éléments (180, 182) à déclic, disposés sur le bloqueur de crantage (152), entrent respectivement en liaison avec des tétons d'arrêt (160, 162) dans la première ou la seconde position.

18. Instrument médical selon la revendication 17, **caractérisé par le fait que**, dans la seconde position, l'élément (182) à déclic entre en liaison d'arrêt avec le téton de crantage (160) qui coopère avec l'organe (24) à crans d'arrêt.

19. Instrument médical selon la revendication 17 ou 18, **caractérisé par le fait que** les éléments (180, 182) à déclic sont disposés sur un gradin (176) s'engageant dans une rainure périphérique (178) pratiquée dans la partie mobile de préhension (20).

20. Instrument médical selon l'une des revendications 1 à 19, **caractérisé par le fait que** l'organe (24) à crans d'arrêt est réalisé sous la forme d'un levier apte à pivoter, précontraint en direction de la partie mobile de préhension (20) et articulé sur la poignée (18).

21. Instrument médical selon l'une des revendications 1 à 20, **caractérisé par le fait que** l'organe (24) à crans d'arrêt est pourvu d'un appendice (25) par l'intermédiaire duquel ledit organe (24) à crans d'arrêt peut être dissocié d'avec la partie mobile de préhension (20).

22. Instrument médical selon la revendication 21, **caractérisé par le fait que** l'appendice (25) revêt la forme d'un arc de cercle dans lequel peut être engagé un doigt d'une main ayant saisi la poignée (18).
